# EUROPEAN PATENT SPECIFICATION

(11) **EP 0 737 462 B1**
(45) Date of publication and mention of the grant of the patent: **20.09.2000**
(21) Application number: 95830145.9
(22) Date of filing: 11.04.1995
(51) Int. Cl.: A61F 13/15

(54) **Sheet material for the production of sanitary napkins and the like and absorbent products made with the said material**
Material zum Herstellen von Monatsbinden und absorbierende Gegenstände, die mit diesem Material hergestellt sind
Matériau pour la fabrication de serviettes hygiéniques et produits absorbants fabriqués avec un tel matériau

(43) Date of publication of application: 16.10.1996
(73) Proprietor: PANTEX S.r.l., I-51031 Agliana, Pistoia (IT)
(72) Inventor: Giacometti, Claudio, I-51030 Pieve a Celle, Pistoia (IT)
(74) Representative: Mannucci, Gianfranco, Dott.-Ing.

(56) References cited:
- FR-A- 2 319 434
- GB-A- 2 171 016
- US-A- 3 665 921
- US-A- 3 945 386
- US-A- 3 967 623
- US-A- 5 171 238
- DATABASE WPI Section Ch, Week 8505 Derwent Publications Ltd., London, GB; Class A96, AN 85-027794 & JP-A-59 222 330 (NITTO ELECTRIC IND KK) , 14 December 1984

## Description

The present invention relates to a sheet material, preferably a plastic film, particularly suitable for the manufacture of absorbent products, such as sanitary napkins in particular but also absorbent products of other types, such as baby diapers and incontinence pads.

The present invention also relates to an absorbent product made using the said sheet material.

Sanitary napkins, baby diapers, incontinence pads and the like are currently produced using an impermeable backsheet, an intermediate absorbent mass and a topsheet that is permeable to fluids (at least in the central zone). The backsheet and topsheet are joined along the edge of the product by any suitable method, such as welding, gluing, etc.

The structure of the topsheet must permit fluid to pass rapidly from the outer to the inner surface, i.e. into the absorbent mass. It must also prevent the fluid passing back to the outside, even when the absorbent mass is compressed, for example by the body weight of the user, and must enable a certain distance to be maintained between the skin of the user (which is in contact with the outer surface of the topsheet) and the absorbent mass inside. Finally, because the topsheet comes into contact with the skin, it is advisable that it should be pleasing to the touch and have a textile handle.

### State of the art

The topsheet is made of a nonwoven fabric or, according to a more recent technique, a perforated plastic film.

The perforated plastic film has the disadvantage of being very thin and especially of being unpleasant to the touch, unlike nonwoven fabric.

To give the plastic film a "textile" exterior appearance and handle and thereby eliminate, or at least reduce, the unpleasing appearance and handle resulting from its shininess and smoothness, numerous processes of varying complexity have been developed for treating the surface of the film that are intended not only to create suitable perforations in the film to render it permeable to fluids, but also to improve its aesthetic, visual and tactile properties. In addition, these processes tend to perforate the film by deforming it, so that its overall thickness is greater than that of the unperforated film. To this end the film is subjected to cold or hot plastic deformation during the perforation operation so that in the zones surrounding the holes flaps of plastic material are created that stand up from the surface of the film. This is known as three-dimensional film, i.e. it has a depth perpendicular to the surface of the film that is much greater than the depth of the unprocessed film.

The various processes proposed for the surface treatment and perforation of the plastic film include, for example, those described in USA patents Nos. 3,484,835, 3,911,187, 3,950,480, 3,957,414 and 4,151,240. Among the most recent solutions, USA patent No. 4,342,314 describes a device and a process for the perforation of a plastic film intended for use in the production of sanitary napkins and baby diapers in which a vacuum roll, with one wall defined by a complex multilayered structure, is used to emboss a plastic film under vacuum. The structure of the roll is designed so that the plastic film assumes an almost fibrous surface structure and so acquires tactile properties very similar to those of fabric. Improvements to this process are described in WO 93/12749. Similarly with the aim of obtaining an air-permeable plastic film with surface properties similar to those of a fabric, USA patent No. 4,463,045 describes a method of perforating the plastic film and at the same time applying a design to the surface of the film, thereby reducing the shininess typical of plastic material.

All the methods of perforation and surface treatment described in the earlier patents cited above are highly complex and difficult to carry out and require expensive machinery. In addition, the results obtained in terms of the tactile properties of the product are unsatisfactory.

EP-A-0,598,970 describes an extremely simple and effective new method of perforating a laminated material and in particular a plastic film, for the purposes described above, using two rolls, one with projections and one that is smooth. The film acquires depth, permeability and surface treatment by virtue of a simple difference in the peripheral speed of the two rolls.

### Objects of the invention

The object of the present invention is to propose a new type of sheet material for use in the production of sanitary napkins, baby diapers, incontinence pads and the like that has even better qualities than the traditional products.

More particularly, the present invention proposes the use of a sheet material, and in particular a plastic film for the production of absorbent articles, whose visual and tactile surface properties are even closer to those of a piece of fabric, whilst retaining all the typical advantages of the plastic film, especially the advantages in terms of providing a protective layer between the user's body and the absorbent mass inside the product, rapid passage of fluid from the outside to the inside of the absorbent product, and preventing fluid passing back from the inside to the outside of the product.

Further objects and advantages of the present invention will become apparent to experts in the field on reading the text below.

### Brief description of the invention

In essence the invention relates to the use of a sheet material to cover the outside of an absorbent product according to claim 1. At least one portion of the surface of the said sheet material bears a layer of fibres applied by flocking, i.e. anchored to the surface of the sheet material by a layer of resin or other adhesive and aligned substantially at right angles to the surface of the said material.

From JP-A-59 222 330 an absorbent article including a fairly thick elastomer provided with a pile of flocked fibers is known. However in this prior art reference the flocked fibers form an absorbent mass.

The sheet material can in principle also be a nonwoven fabric but is preferably a plastic film of the type usually used to cover sanitary napkins, diapers and similar products. In this way, all the advantages of the plastic film are retained while its disadvantages are eliminated, in particular its unpleasant tactile properties when the product is in contact with the skin.

The technique of flocking is known per se in the textile industry for the production of mock velvet. In these applications, the fibres are applied to a laminar support made of fabric or even nonwoven fabric that is fairly thick.

In principle, the flocked sheet material can be used to cover either the back or the top of an absorbent or similar product. If used as a backsheet it will not be perforated. However, the major advantages are achieved by using the flock-covered plastic film as the topsheet in an absorbent product. In this case the plastic film will be fully or partially perforated to make it permeable to fluids.

The plastic film can be perforated by any known method, for example any of the methods described in the patents cited above, and will preferably be perforated before undergoing the flocking process. It can also be embossed before being perforated.

The invention also relates, in particular, to an absorbent product of the type comprising an impermeable backsheet, a topsheet that is permeable to fluids and an absorbent mass enclosed between the said topsheet and the said backsheet. According to the present invention, at least the topsheet of the absorbent product is made of a sheet material, to at least one portion of the external surface of which fibres are applied by flocking, i.e. are attached to the external surface by a layer of polymerized resin or other adhesive and aligned at right angles to the surface of the sheet material.

Depending on the structure of the sheet material, fibres with hydrophobic or hydrophilic properties can be used. If, for example, the sheet material has a perforated zone free of flocked fibres and an unperforated zone covered with fibres, the latter will advantageously be hydrophobic so as to repel the fluid and direct it towards the zone which is free of fibres, where the perforations will permit its rapid downflow into the absorbent mass underneath. If the surface of the sheet material is either completely or partially perforated and is completely covered with fibres applied by flocking, then these fibres should be hydrophilic so as not to impede the downflow of the fluid from the top surface of the product into the absorbent mass inside it. The resin used to anchor the fibres to the sheet material can also be selected to suit the type of application. A closed-cell resin is preferable for the application of hydrophobic fibres to zones where rapid "horizontal" removal of the fluid is required, i.e. removal along the surface towards perforated zones that permit the downward flow of the fluid in a "vertical" direction, i.e. through the sheet material into the interior of the absorbent product. An open-cell resin is preferable in the case where the fibres are applied to the zone where the sheet material is perforated and is designed to allow the downflow of the fluid from the outer surface to the interior of the absorbent product.

The length of the fibres can be selected, for example, from a range of 0.3 to 2 mm and preferably of 0.5 to 1.5 mm. The count can vary between 0.3 and 5 denier and preferably between 2.7 and 45 tex (0.5 and 3 denier). Examples of suitable fibres are those of the cellulose, polyamide, polypropylene, polyester or equivalent type.

### Brief description of the drawings

The invention will be better understood from the description and attached drawing, which shows a practical nonlimiting embodiment of the said invention. In the drawing:
Fig. 1 shows a diagram of a flocking plant;
Fig. 1A shows an enlarged front view of a possible form of doctor blade suitable for use in the plant in Fig. 1;
Fig. 2 shows a piece of plastic film with flocked strips;
Fig. 3 shows a sanitary napkin made with the film shown in Fig. 2;
Figs 4A and 4B show two cross-sections of two different embodiments of the sanitary napkin shown in Fig. 3;
Fig. 5 shows a cross-section, similar to those in Figs 4A and 4B, of a third embodiment of a sanitary napkin;
Figs 6 and 7 show diagrammatic enlargements of two cross-sections of two films according to the invention.

### Detailed description of the invention

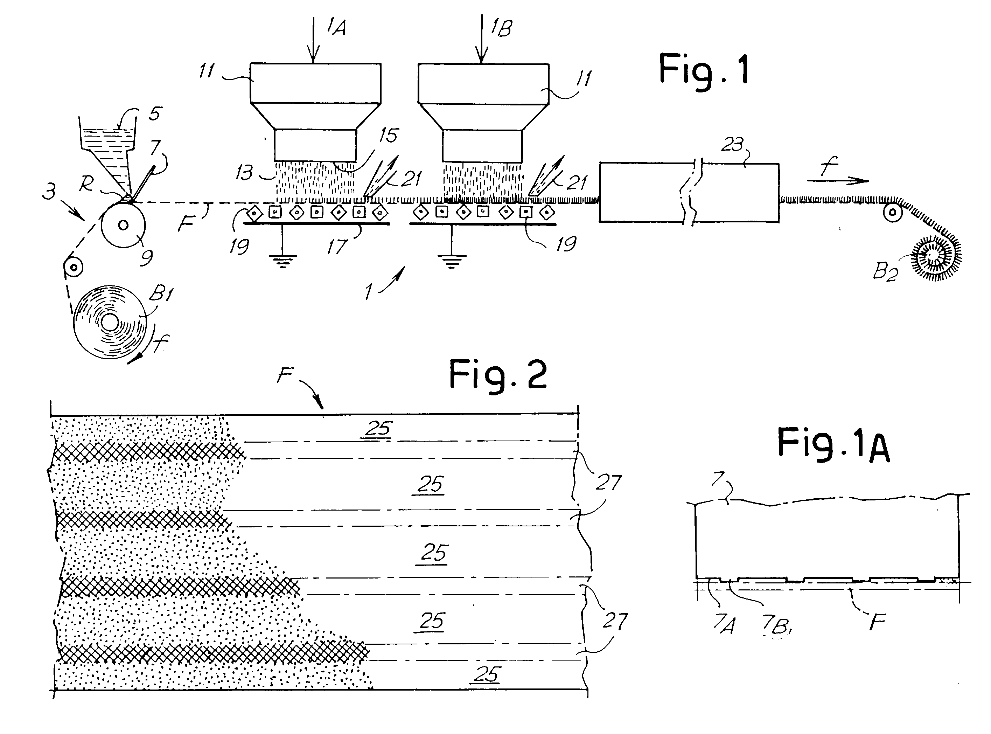

Fig. 1 shows a highly diagrammatic representation of a plant for the production of a flocked film according to the invention. Plants of this type are known per se and are widely used in the manufacture of mock velvets. A plant of this type can be adapted for the production of the film according to the present invention. The structure of the plant is described briefly, without giving details of its construction, which will be known to experts in the field.

B1 is a reel of plastic film F to be processed, which is unwound in the direction of the arrow f and is fed into a flocking machine 1. Upstream of the flocking machine 1 is a spreading station 3, in which a nozzle 5 or other appropriate device applies a polymerizable resin R to the upper surface of the film F. Downstream of the nozzle 5 is a doctor blade 7 which distributes the resin over the film F. The doctor blade can have a smooth edge positioned a preset distance from the surface of the film and parallel to it to ensure that a uniform layer of constant thickness is spread on the film. Conversely, a doctor blade with a toothed profile can be used as shown diagrammatically in Fig. 1A, which shows a front view of a portion of the doctor blade. By positioning the doctor blade so that the teeth 7B touch the surface of the film F, strips of resin will be applied to the latter that are of uniform thickness (roughly equal to the depth of the teeth 7B) and have a width, in the tranverse direction to the direction of movement of the film, equal to the length of the indented portions 7A of the doctor blade between one tooth and the next. The doctor blade 7 works in conjunction with a counter roller 9 or with another appropriate counter device, for example a conveyor belt or similar element on which the film F rests.

The flocking machine illustrated in Fig. 1 is a dual-station version but the use of a single-station version or a version with more than two stations is not excluded. Each station 1A, 1B has a metering device 11 which distributes an adjustable quantity of fibres 13 onto the film F below. The area in which the fibres 13 fall is enclosed in an electrostatic field whose force lines are vertical and perpendicular to the film F. The electrostatic field is created by two electrodes 15 and 17 situated above and below the film F. This means that the orientation of the fibres when they become anchored to the layer of resin spread on the upper surface of the film is the same as that of the force lines of the electrostatic field. A vibrator 19 situated below the film keeps the latter in a state of high frequency vibration to ensure correct distribution of the fibres and removal of the fibres that are not correctly anchored in the resin. The latter are collected by a suction device 21.

Downstream of the flocking machine, the film passes through a drying chamber 23 where the resin is polymerized. On emerging from the chamber the film is then rewound onto a reel B2.

The film F on the reel B1 can be a previously perforated film, or the spreading station 3, the flocking machine 1 and the chamber 23 can be installed downstream of a section of processing line in which an unperforated film is continuously perforated. In the latter case, the processing takes place in a single line, starting with an intact and basically two-dimensional film and ending with a perforated film with a three-dimensional, flocked structure.

The possibility of the film being perforated after the flocking has been carried out is not excluded. However, this can be problematic since the heat or pressure applied to the film to create the perforations can damage the flocked fibres.

The film can be perforated over its entire surface or only in certain zones, as described in EP-A-0,598,970. If the flocked film is to be used as the backsheet of an absorbent product it should not be perforated.

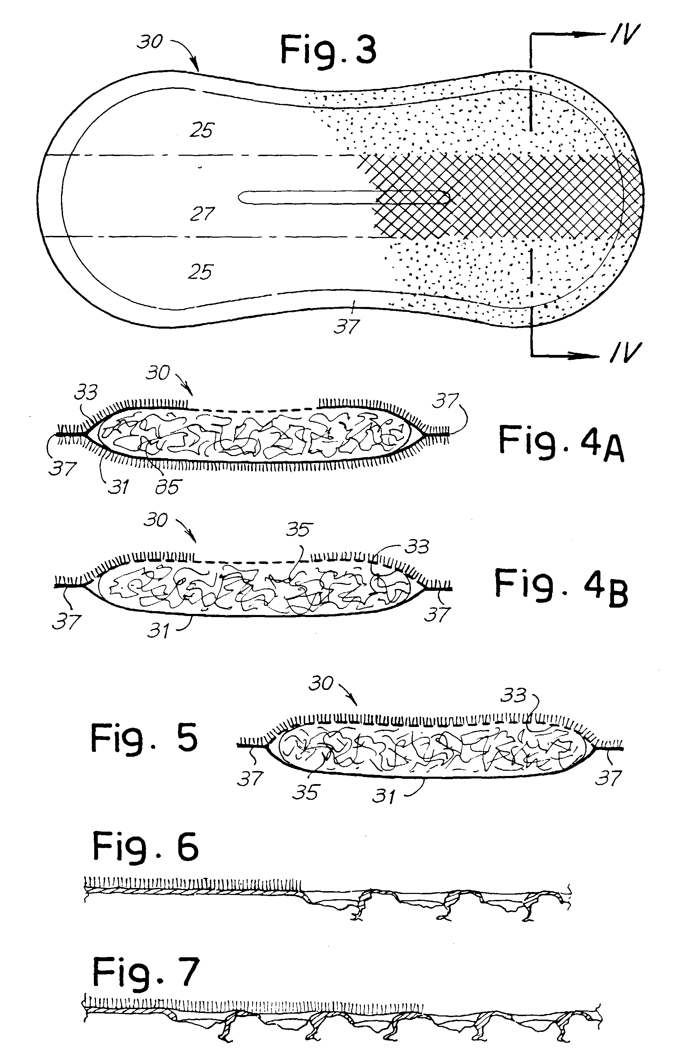

Fig. 2 shows a portion of film F obtained using a doctor blade 8 of the type shown in Fig. 1A. The film has strips 25 to which are anchored the fibres 13 and strips 27 bearing no fibres. Over the area of at least the strips 27 the film is perforated. A film of this type can be used in the production of sanitary napkins as illustrated in Figs 3, 4A, 4B and 5. 31 indicates the backsheet of the sanitary napkin 30 and 33 the topsheet. Between the two layers is the absorbent mass 35. The two layers 31 and 33 are joined along the edge 37 of the sanitary napkin. As is clearly visible in Fig. 3, the topsheet 33 of the sanitary napkin is made with a film F of the type with flocked strips, as illustrated in Fig. 2. The perforated and unflocked strip 27 of the film F forms the central zone of the sanitary napkin 30 with the flocked, preferably unperforated, strips 25 on either side of this.

In this way the plastic surface of the film F comes into contact with the body only in the central zone, while the lateral zones have a layer of flocked fibres and the skin is therefore in contact with the fibres. If the flocked fibre is treated to make it hydrophobic, this will cause the fluid to flow along the surface of the film towards the central zone, where it will pass through the holes in the film and rapidly be absorbed by the absorbent mass inside the product. As is clearly visible in Figs 4A and 4B, the film forming the topsheet 33 can be perforated either in the central zone alone (Fig. 4A) or over the entire surface (Fig. 4B). The second solution may be preferable if the said perforated film is to be used as a semiprocessed product for various applications, for example for sanitary napkins and baby diapers.

The backsheet 31 of the sanitary napkin 30 does not come into contact with the skin and can therefore be made of ordinary plastic film--(Figs 4B and 5). The use of a flocked film for the backsheet 31 as well (Fig. 4A) gives both surfaces of the sanitary napkin a textile handle.

Figs 6 and 7 show two enlarged, diagrammatic cross-sections of a flocked film F. Fig. 6 shows a piece of film with perforated strips and flocked strips. It has an unperforated portion to which the flocked fibre is applied and a perforated portion with no fibres. Fig. 7, by contrast, shows a partially perforated and partially unperforated film with fibres applied over the entire surface. In this case a roughly uniform layer of flocked fibre is formed over the entire surface of the film.

The drawings are intended to show only one embodiment given by way of a practical demonstration of the invention, since the said invention can vary in shape and disposition without thereby departing from the scope of the underlying concept of the said invention, as defined in the attached claims. Any reference numbers given in the attached claims are intended to facilitate the reading of the claims with reference to the description and the drawings and do not limit the scope of the protection afforded by them.

## Claims

1. Use of a sheet material (F) including a first surface and a second surface, at least a portion of said first surface being provided with a layer of fibres (13) applied by flocking, for covering the outside of an absorbent product, said first surface and the layer of flocked fibres (13) being arranged on the outside of said absorbent product.

2. Use according to claim 1, wherein said absorbent product includes a backsheet (31), a liquid pervious topsheet (33), an absorbent mass (35) enclosed between said topsheet and said backsheet, and wherein said sheet material (F) is used as a topsheet.

3. Use according to claim 1 or 2, wherein said sheet material (F) is a plastic film.

4. Use according to claim 2 or 3, wherein said sheet material is perforated.

5. Use according to claim 4, wherein said sheet material (F) is deformed in the perforated zone and has an overall thickness greater than that of the unperforated sheet material.

6. Use according to claim 4 or 5, wherein at least one portion (27) of said first surface bears no flocked fibres (13) and adjacent portions (25) of said first surface have a layer of flocked fibres (13), at least said portion that bears no flocked fibres being perforated.

7. An absorbent product comprising an impermeable backsheet (31), a liquid pervious topsheet (33) and an absorbent mass (35) enclosed between said topsheet and said backsheet, characterised in that at least said topsheet (33) is made of a sheet material (F) to at least one portion of the external surface of which a layer of flocked fibres (13) is applied.

8. Absorbent product according to claim 7, characterised in that said topsheet (33) is an at least partially perforated plastic film, the overall thickness of the perforated area of said film being greater than that of the unperforated film.

9. Absorbent product according to claim 8, characterised in that said topsheet (33) has a central strip (27) free of flocked fibres, and two lateral strips (25) to which a layer of flocked fibres is applied.

10. Absorbent product according to claim 9, characterised in that said fibres (13) are hydrophobic fibres.

11. Absorbent product according to claim 10, characterised in that said plastic film is entirely perforated and that at least one portion of its surface bears a layer of flocked fibres (13).

12. Absorbent product according to one or more of claims 7 to 11, characterised in that the backsheet (31) has a covering of flocked fibres (13) on its external surface.

13. Absorbent product according to one or more of claims 7 to 12, characterised in that said fibres (13) are between approximately 0.3 and 2 mm long and have a count of between approximately 2.7 tex (0.3 denier) and 45 tex (5 denier).

## Patentansprüche

1. Verwendung eines Folienmaterials (F) mit einer ersten Oberfläche und einer zweiten Oberfläche, wobei zumindest ein Teil der ersten Oberfläche mit einer Schicht aus Fibern (13) versehen ist, die durch Beflockung angebracht sind, zum Abdecken der Außenseite eines absorbierenden Produkts, wobei die erste Oberfläche und die Schicht aus beflockten Fibern (13) an der Außenseite des absorbierenden Produkts angeordnet sind.

2. Verwendung nach Anspruch 1,
wobei das absorbierende Produkt eine Rückfolie (31), eine flüssigkeitsdurchlässige Oberfolie (33), eine absorbierende Masse (35), die zwischen der Oberfolie und der Rückfolie angeordnet ist, aufweist, und wobei das Folienmaterial (F) als Oberfolie dient.

3. Verwendung nach Anspruch 1 oder 2,
wobei das Folienmaterial (F) ein Kunststoffilm ist.

4. Verwendung nach Anspruch 2 oder 3,
wobei das Folienmaterial perforiert ist.

5. Verwendung nach Anspruch 4,
wobei das Folienmaterial (F) in der perforierten Zone verformt ist und eine Gesamtdicke aufweist, die größer ist als die des unperforierten Folienmaterials.

6. Verwendung nach Anspruch 4 oder 5,
wobei zumindest ein Teil (27) der ersten Oberfläche keine beflockten Fibern (13) trägt und angrenzende Teile (25) der ersten Oberfläche eine Schicht aus beflockten Fasern (13) aufweist, wobei zumindest der Teil, der keine beflockten Fibern trägt, perforiert ist.

7. Absorbierendes Produkt mit einer undurchlässigen Rückfolie (31), einer flüssigkeitsdurchlässigen Oberfolie (33) und einer absorbierenden Masse (35), die zwischen der Oberfolie und der Rückfolie eingeschlossen ist,
dadurch **gekennzeichnet,** daß zumindest die Oberfolie (33) aus einem Folienmaterial (F) hergestellt ist, wobei zumindest auf einen Teil ihrer externen Oberfläche eine Schicht aus beflockten Fibern (13) aufgebracht ist.

8. Absorbierendes Produkt nach Anspruch 7,
dadurch **gekennzeichnet,** daß die Oberfolie (33) ein zumindest teilweise perforierter Kunststoffilm ist, wobei die Gesamtdicke des perforierten Bereichs des Films größer ist als die des unperforierten Films.

9. Absorbierendes Produkt nach Anspruch 8,
dadurch **gekennzeichnet,** daß die Oberfolie (33) einen zentralen Streifen (27) aufweist, der frei von beflockten Fibern ist, und zwei seitliche Streifen (25), in denen beflockte Fibern angebracht sind.

10. Absorbierendes Produkt nach Anspruch 9,
dadurch **gekennzeichnet,** daß die Fibern (13) hydrophobe Fibern sind.

11. Absorbierendes Produkt nach Anspruch 10,
dadurch **gekennzeichnet,** daß der Kunststofffilm vollständig perforiert ist und daß zumindest ein Teil seiner Oberfläche eine Schicht aus beflockten Fibern (13) trägt.

12. Absorbierendes Produkt nach einem oder mehreren der Ansprüche 7 bis 11,
dadurch **gekennzeichnet,** daß die Rückfolie (31) eine Abdeckung aus beflockten Fibern (13) auf ihrer externen Oberfläche aufweist.

13. Absorbierendes Produkt nach einem oder mehreren der Ansprüche 7 bis 12,
dadurch **gekennzeichnet,** daß die Fibern (13) zwischen etwa 0,3 und 2 mm lang sind und eine Zahl von näherungsweise 2,7 tex (0,3 denier) und 45 tex (5 denier) aufweisen.

## Revendications

1. Utilisation d'un matériau en feuilles (F) comprenant une première surface et une deuxième surface, au moins une partie de la première surface étant pourvue d'une couche de fibres (13) appliquées par flocage, destiné à recouvrir l'extérieur d'un produit absorbant, la première surface et la couche de fibres floquées (13) étant prévues sur l'extérieur du produit absorbant.

2. Utilisation selon la revendication 1, dans laquelle le produit absorbant comprend une feuille de revers (31), une feuille supérieure (33) perméable aux liquides, une masse absorbante (35) enfermée entre la feuille supérieure et la feuille de revers, et dans laquelle le matériau en feuilles (F) est utilisé comme feuille supérieure.

3. Utilisation selon la revendication 1 ou 2, dans laquelle le matériau en feuilles (F) est une pellicule plastique.

4. Utilisation selon la revendication 2 ou 3, dans laquelle le matériau en feuilles est perforé.

5. Utilisation selon la revendication 4, dans laquelle le matériau en feuilles (F) est déformé dans la zone perforée et présente une épaisseur globale supérieure à celle du matériau en feuilles non perforé.

6. Utilisation selon la revendication 4 ou 5, dans laquelle au moins une partie (27) de la première surface ne porte pas de fibres floquées (13) et les parties adjacentes (25) de la première surface présentent une couche de fibres floquées (13), au moins la partie qui ne porte pas de fibres floquées étant perforée.

7. Produit absorbant comprenant une feuille de revers (31) imperméable, une feuille supérieure (33) perméable aux liquides et une masse absorbante (35) enfermée entre la feuille supérieure et la feuille de revers, caractérisé en ce qu'au moins la feuille supérieure (33) est constituée en matériau en feuilles (F), sur au moins une partie de la surface externe duquel une couche de fibres floquées (13) est appliquée.

8. Produit absorbant selon la revendication 7, caractérisé en ce que la feuille supérieure (33) est une pellicule plastique au moins partiellement perforée, l'épaisseur globale de la zone perforée de la pellicule étant supérieure à celle de la pellicule non perforée.

9. Produit absorbant selon la revendication 8, caractérisé en ce que la feuille supérieure (33) comprend une bande centrale (27) dépourvue de fibres floquées, et deux bandes latérales (25) sur lesquelles une couche de fibres floquées est appliquée.

10. Produit absorbant selon la revendication 9, caractérisé en ce que les fibres (13) sont des fibres hydrophobes.

11. Produit absorbant selon la revendication 10, caractérisé en ce que la pellicule plastique est entièrement perforée et en ce qu'au moins une partie de sa surface porte une couche de fibres floquées (13).

12. Produit absorbant selon l'une ou plusieurs des revendications 7 à 11, caractérisé en ce que la feuille de revers (31) comprend un revêtement de fibres floquées (13) sur sa surface externe.

13. Produit absorbant selon l'une ou plusieurs des revendications 7 à 12, caractérisé en ce que les fibres (13) sont d'une longueur comprise entre approximativement 0,3 et 2 mm, et leur titre est compris entre approximativement 2,7 tex (0,3 denier) et 45 tex (5 deniers).
